# EUROPEAN PATENT SPECIFICATION

(11) **EP 1 784 152 B1**
(45) Date of publication and mention of the grant of the patent: **03.08.2011**
(21) Application number: 05857287.6
(22) Date of filing: 31.08.2005
(51) Int. Cl.: A61F 13/494

(54) **EDGE BAND FOR ABSORBENT ARTICLE AND METHOD FOR MAKING**
EINLEIMER FÜR SAUGFÄHIGE ARTIKEL UND HERSTELLUNGSVERFAHREN
BANDE PERIPHERIQUE POUR ARTICLE ABSORBANT ET PROCEDE DE FABRICATION

(30) Priority: 01.09.2004 US 932199
(43) Date of publication of application: 16.05.2007
(73) Proprietor: The Procter & Gamble Company, Cincinnati, OH 45202 (US)
(72) Inventor: SCHNEIDER, Uwe, Cincinnata, Ohio 45215 (US); BRIDGES, Russell, Pearce, Cincinnati, OH 45230 (US)
(74) Representative: Borbach, Markus
(86) International application number: PCT/US2005/030908
(87) International publication number: WO 2006/028832

(56) References cited:
- EP-A- 0 528 282
- WO-A-97/21410
- US-A- 5 620 431

## Description

### FIELD OF THE INVENTION

This invention relates to edge bands on absorbent articles, and more particularly to discrete edge bands, such as, discrete elasticized edge bands on disposable absorbent articles, such as diapers.

### BACKGROUND OF THE INVENTION

Infants and other incontinent individuals wear disposable absorbent articles such as diapers to receive and contain urine and other body exudates. Absorbent articles function both to contain the discharged materials and to isolate these materials from the body of the wearer and from the wearer's garments and bed clothing. Disposable absorbent articles having many different basic designs are known to the art.

A typical absorbent article has a chassis that includes a liquid pervious topsheet, and a liquid impervious backsheet joined to the topsheet. An absorbent core is typically positioned between the topsheet and the backsheet. The chassis has a generally elongated shape, which can be rectangular or hourglass shaped. The long dimension of the chassis defines two opposed edges referred to as the longitudinal edges. A portion of each longitudinal edge defines the leg opening of the diaper when worn.

To better contain body exudates, absorbent articles often have elasticized leg openings, referred to as leg cuffs that are designed to fit snugly about the legs of the wearer. Leg cuffs can be made by simply placing tensioned elastic strands along the longitudinal edges, for example. When contracted the elastic strands form gathers of material. Leg cuffs, as opposed to barrier cuffs that are generally disposed inboard, that is, toward the center of the diaper and away from the longitudinal edges of the diaper chassis, can include a portion of the longitudinal edge of the diaper chassis. This longitudinal edge is often unsightly, that is, it appears as an unfinished edge that may have a rather rough appearance. The appearance is important, as consumer perception of softness and comfort are significant considerations for commercial success in the disposable absorbent article field.

In addition to the appearance of leg cuffs in absorbent articles, leg cuffs are relatively costly. In particular, for elasticized leg cuffs, there is a need for elastic in the portion of the longitudinal edge of the chassis that defines the leg opening. However, due to the nature of commercial production of disposable absorbent garments, it is not feasible to apply discrete segments of a material, such as a patch or a strand of elastic material, to a discrete portion of the diaper chassis. For example, it is very difficult to cut a strand of elastic to a length shorter than the long dimension of the chassis and affix it in a predetermined location on the chassis, all at high speed in a repeating manner. For this reason, leg cuff (as well as barrier cuff) elastics are often applied as continuous strands that extend the entire length of the long dimension of the chassis, generally near the longitudinal edges. However, the extra elastic, beyond the portion of the longitudinal edges that define the leg opening, is non-value-added material content.

Accordingly, it would be desirable to have a disposable absorbent article having a leg cuff that has a finished, comfortable appearance.

Additionally, it would be desirable to have a method for making a leg cuff having a finished appearance in a commercially viable manner.

Further, it would be desirable to have a disposable absorbent article with a leg cuff having a finished appearance, with the finished appearance limited to the portion of the longitudinal edge of the chassis that defines a leg opening when worn.

Finally, it would be desirable to have a commercially-viable method of making a disposable absorbent article with a leg cuff having a finished appearance, with the finished appearance limited to the portion of the longitudinal edge of the chassis that defines a leg opening when worn.

### BRIEF SUMMARY OF THE INVENTION

In accordance with a first aspect of the present invention, a disposable diaper is provided. The disposable diaper comprises a topsheet, a backsheet, and an absorbent core disposed between the topsheet and the backsheet, the topsheet and the backsheet defming a periphery and longitudinal edges, portions of the longitudinal edges defining leg openings when the disposable diaper is worn, the disposable diaper comprising on each longitudinal edge a discrete edge band joined to the longitudinal edges at least at the portions of the longitudinal edges defining leg openings when the disposable diaper is worn, wherein the discrete edge bands do not extend the entire length of the longitudinal edge of the disposable diaper.

In accordance with a second aspect of the present invention, apparatus for affixing a discrete edge band onto a portion of a longitudinal edge of a continuous web is provided. The apparatus having an axis of rotation, and comprises
a) a first web surface disposed at a first radial distance from the axis;
b) an edge band placer comprising a second web surface disposed at a second radial distance from the axis, the second radial distance being variable between a first minimum distance and a second maximum distance, wherein at the second maximum distance the second radial distance is substantially equal to the first radial distance;
c) the edge band placer adapted to receive edge band material on the second web surface at the first minimum distance and to affix the edge band material as a discrete edge band onto a portion of the longitudinal edge of the continuous web at the second maximum distance.

In accordance with a third aspect of the present invention, a method of affixing a discrete edge band onto a predetermined portion of a longitudinal edge of a continuous web is provided. The method comprises the steps of:
a) providing the continuous web;
b) providing a continuous web of edge band material;
c) providing an edge band placer that is translatable radially outward with respect to an axis of rotation between a first minimum distance and a second maximum distance;
d) receiving a portion of the continuous web of edge band material upon a surface of the edge band placer;
e) applying adhesive to either of the portion of the continuous web of edge band material or the continuous web in a predetermined location;
f) cutting off the portion of the continuous web of edge band material from the continuous web of edge band material;
g) retaining the portion from the continuous web of edge band material upon the surface of the edge band placer;
h) translating the edge band placer from the first minimum distance to the second maximum distance; and
i) joining the portion of the continuous web of edge band material to the predetermined portion of the longitudinal edge of the continuous web.

Alternatively, step (e) may also precede step (d).

### BRIEF DESCRIPTION OF THE DRAWINGS

While the specification concludes with claims particularly pointing out and distinctly claiming the subject matter that is regarded as the present invention, it is believed that the invention will be more fully understood from the following description taken in conjunction with the accompanying drawings. None of the drawings are necessarily to scale.
FIG. 1 is a perspective view of one embodiment of an absorbent article of the present invention.
FIG. 2a is a cross-sectional view of a portion of an edge band portion of the absorbent article shown in Figure 1, wherein the edge band is joined to the topsheet.
FIG. 2b is a cross-sectional view of a portion of an edge band portion of the absorbent article similar to FIG. 2a, wherein the edge band is joined to the backsheet.
FIG. 3a is a cross-sectional view of an alternative embodiment of an edge band portion of an absorbent article, wherein the edge band is joined to the topsheet and includes a fold which is also joined to the topsheet.
FIG. 3b is a cross-sectional view of an alternative embodiment of an edge band portion of an absorbent article, wherein the edge band is joined to the backsheet and includes a fold which is also joined to the backsheet.
FIG. 4a is a cross-sectional view of an alternative embodiment of an edge band portion of an absorbent article, wherein the edge band is joined to the topsheet and includes a fold which is outboard of the topsheet.
FIG. 4b is a cross-sectional view of an alternative embodiment of an edge band portion of an absorbent article, wherein the edge band is joined to the backsheet and includes a fold which is outboard of the backsheet.
FIG. 5 is a cross-sectional view of an alternative embodiment of an edge band portion of an absorbent article, wherein the edge band includes a barrier cuff.
FIG. 6 is a cross-sectional view of an alternative embodiment of an edge band portion of an absorbent article, wherein the edge band includes a barrier cuff and an outboard fold.
FIG. 7 is a cross-sectional view of an alternative embodiment of an edge band portion of an absorbent article, wherein the edge band includes a barrier cuff and an inboard fold.
FIG. 8 is an exemplary, cross-sectional view of a portion of an edge band portion of an absorbent article shown in FIG. 2a, wherein the edge band includes elastics.
FIG. 9 is a perspective schematic representation of an apparatus of the present invention for making an absorbent article of the present invention.
FIG. 10 is a schematic representation of an apparatus of the present invention for making an absorbent article of the present invention.
FIG. 11 is a schematic cross-sectional view of an apparatus of the present invention.

### DETAILED DESCRIPTION OF THE INVENTION

### Definitions:

As used herein, the term "disposed" is used to mean that an element(s) of the diaper is formed (joined and positioned) in a particular place or position as a unitary structure with other elements of the diaper or as a separate element joined to another element of the diaper.

The term "joined" herein encompasses configurations whereby a material or component is secured directly or indirectly (by one or more intermediate members) to another material or component. An example of indirect joining is an adhesive. Direct bonding includes heat in conjunction with or alternatively pressure bonding. Joining may include any means known in the art including, for example, adhesives, heat bonds, pressure bonds, ultrasonic bonds, and the like.

The term "nonwoven" refers herein to a material made from continuous (long) filaments (fibers) and/or discontinuous (short) filaments (fibers) by processes such as spunbonding, meltblowing, and the like. Nonwovens do not have a woven or knitted filament pattern. Nonwovens are typically described as having a machine direction and a cross direction. The machine direction is the direction in which the nonwoven is manufactured. The cross direction is the direction perpendicular to the machine direction. Nonwovens are typically formed with a machine direction that corresponds to the long or rolled direction of fabrication. The machine direction is also the primary direction of fiber orientation in the nonwoven.

As used herein, the term "elastic" or "elastomeric" refers to any material which, upon application of a biasing force, is stretchable, that is, elongatable, at least about 60 percent (i.e., to a stretched, biased length, which is at least about 160 percent of its relaxed unbiased length), and which, will recover at least 55 percent of its elongation upon release of the stretching, elongation force. A hypothetical example would be a one (1.0) cm sample of a material which is elongatable to at least 1.60cm, and which, upon being elongated to 1.60cm and released, will recover to a length of not more than 1.27cm. Many elastic materials may be elongated by more than 60 percent (i.e., much more than 160 percent of their relaxed length), for example, elongated 100 percent or more, and many of these materials will recover to substantially their initial relaxed length, for example, to within 105 percent of their initial relaxed length, upon release of the stretch force. Such materials are referred to herein as "highly elastic".

As used herein, the term "nonelastic" refers to any material that does not fall within the definition of "elastic" (or "elastomeric") or "highly elastic" above.

As used herein, the term "extensible" refers to any material that, upon application of a biasing force, is elongatable, at least about 50% without offering a significant resistance force (less than 10 g/cm) or experiencing catastrophic failure. Catastrophic failure includes substantial tearing, fracturing, rupturing, or other failure in tension such that, if tested in a standard tensile tester, the failure would result in a sudden significant reduction in measured tensile force. As used herein, the term "highly extensible" refers to any material which, upon application of a biasing force, is elongatable, at least about 70%, more preferably at least about 100%, and even more preferably about 120% without offering a significant resistance force (less than 10 g/cm) or experiencing catastrophic failure.

The term "absorbent article" herein refers to devices which absorb and contain body exudates and, more specifically, refers to devices which are placed against or in proximity to the body of the wearer to absorb and contain the various exudates discharged from the body, such as: incontinence briefs, incontinence undergarments, absorbent inserts, diaper holders and liners, feminine hygiene garments and the like.

The term "disposable" is used herein to describe absorbent articles which generally are not intended to be laundered or otherwise restored or reused as absorbent articles (i.e., they are intended to be discarded after a single use and, preferably, to be recycled, composted or otherwise discarded in an environmentally compatible manner).

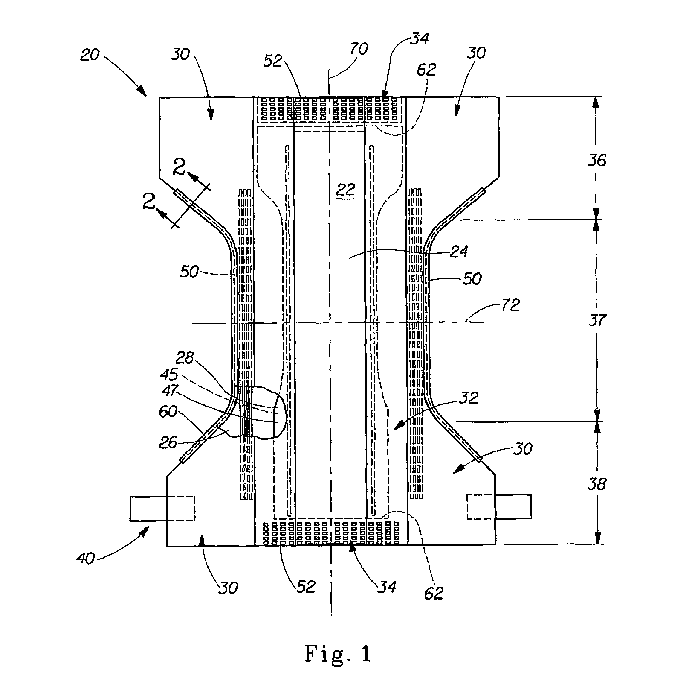

A "unitary" absorbent article refers to absorbent articles that are formed of separate parts united together to form a coordinated entity so that they do not require separate manipulative parts like a separate holder and liner. A preferred embodiment of an absorbent article of the present invention is the unitary disposable absorbent article, diaper 20, shown in FIG. 1. As used herein, the term "diaper" refers to an absorbent article generally worn by infants and incontinent persons about the lower torso, and includes both tape-type diapers (diapers having adhesive tapes, hook and loop fasteners, and the like, that fastened about the waist of the wearer), and pull-on pant-type diapers. The present invention is also applicable to other absorbent articles such as incontinence briefs, incontinence undergarments, absorbent inserts, diaper holders and liners, and the like.

The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the term "pant" is used herein, pants are also commonly referred to as "closed diapers", "prefastened diapers", "pull-on diapers", "training pants" and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

As used herein, the term "discrete edge band" or "edge band" refers to a discrete elongate element that does not wrap about a portion of a longitudinal edge of a diaper, rather is affixed/joined to one side of that portion of the "edge" of the diaper. Edge bands as described herein provide a soft, finished appearance to the portions of the longitudinal edge of a diaper that form the leg openings when worn. In contrast, an "edge seal" refers to a discrete elongate element that does wrap about a portion of a longitudinal edge of a diaper and therefore requires substantially more material than the "edge band" of the present invention.

All measurements are in SI units, unless otherwise specified.

### Descriptions:

FIG. 1 is a plan view of the diaper 20 of the present invention in a flat-out state with portions of the structure being cut away to more clearly show the construction of the diaper 20. The portion of the diaper 20 that faces the wearer is oriented towards the viewer. As shown in FIG. 1, the diaper 20 preferably comprises a liquid pervious topsheet 24; a liquid impervious backsheet 26, an absorbent core 28 which is preferably positioned between at least a portion of the topsheet 24 and the backsheet 26; side panels 30; elasticized leg cuffs 32; and an elastic waist feature 34.

Diaper 20 is shown in FIG. 1 to have a first waist region 36, a second waist region 38 opposed to the first waist region 36 and a crotch region 37 located between the first waist region and the second waist region 38. The periphery of the diaper 20 is defined by the outer edges of the diaper 20 in which the longitudinal edges 50 are oriented generally parallel to the longitudinal centerline 70 of the diaper 20 and the end edges 52 run between the longitudinal edges 50 generally parallel to the transverse centerline 72 of the diaper 20. However, for better fit, longitudinal edges 50 are preferably curved to produce an "hourglass" shape diaper when viewed in the flat-out configuration of FIG. 1. The chassis 22 of the diaper 20 comprises the main body of the diaper 20. The chassis 22 comprises at least the topsheet 24 and the backsheet 26.

For tape-type diapers, that is, diapers intended to be fastened about the wearer by use of an adhesive tape or releasable mechanical fastener, the diaper 20 can have a fastening system generally designated 40, as is commonly known in the art. Once fastened upon the wearer, portions of longitudinal edge 50 define leg openings. For pant-type diapers, first waist region 36 is joined by suitable means to the second waist region 38, as is commonly known in the art, to make a pant-type garment having leg openings defined by the non-joined portions of longitudinal edges 50. Joining of the waist regions can be by thermal bonding, adhesive bonding, or ultrasonic bonding, for example.

While the topsheet 24, the backsheet 26, and the absorbent core 26 may be assembled in a variety of well-known configurations, preferred tape-diaper configurations are described generally in U.S. Pat. No. 3,860,003 entitled "Contractible Side Portions for Disposable Diaper" which issued to Kenneth B. Buell on Jan. 14, 1975; U.S. Pat. No. 5,151,092 issued to Buell on Sep. 9, 1992; and U.S. Pat. No. 5,221,274 issued to Buell on Jun. 22, 1993; and U.S. Pat. No. 5,554,145 entitled "Absorbent Article With Multiple Zone Structural Elastic-Like Film Web Extensible Waist Feature" which issued to Roe et al. on Sep. 10, 1996; U.S. Pat. No. 5,569,234 entitled "Disposable Pull-On Pant" which issued to Buell et al. on Oct. 29, 1996; and U.S. Pat. No. 5,580,411 entitled "Zero Scrap Method For Manufacturing Side Panels For Absorbent Articles" which issued to Nease et al. on Dec. 3, 1996. Preferred pant-type diapers and methods for making suitable side seams are disclosed in U.S. Patent No. 5,569,234 issued to Buell, et al. on October 29, 1996, U.S. Patent No. 5,607,537 issued to Johnson et al. on March 4, 1997, U.S. Patent No. 5,662,638 issued to Johnson et al. on September 2, 1997, and U.S. Patent No. 5,685,874 issued to Buell et al. on November 11, 1997. Preferable seams are disclosed in European Patent Application No. 96118654.1 titled "Thermal Joining of Webs" filed on November 21, 1996 (Christoph J. Schmitz).

The backsheet 26 is generally that portion of the diaper 20 positioned adjacent the garment facing surface 45 of the absorbent core 28 which prevents the exudates absorbed and contained therein from soiling articles which may contact the diaper 20, such as bed sheets and undergarments. In preferred embodiments, the backsheet 26 is impervious to liquids (e.g., urine) and comprises a thin plastic film such as a thermoplastic film having a thickness of about 0.012 mm (0.5 mil) to about 0.051mm (2.0 mils). Suitable backsheet films include those manufactured by Tredegar Industries Inc. of Terre Haute, Ind. and sold under the trade names XI 5306,X10962 and X10964. Other suitable backsheet materials may include breathable materials that permit vapors to escape from the diaper 20 while still preventing exudates from passing through the backsheet 26. Exemplary breathable materials may include materials such as woven webs, nonwoven webs, composite materials such as film-coated nonwoven webs, and micro porous films such as manufactured by Mitsui Toatsu Co., of Japan under the designation ESPOIR NO and by EXXON Chemical Co., of Bay City, Tex., under the designation EXXAIRE. Suitable breathable composite materials comprising polymer blends are available from Clopay Corporation, Cincinnati, Ohio under the name HYTREL® blend P18-3097. Such breathable composite materials are described in greater detail in PCT Application No. WO 95/16746, published on Jun. 22, 1995 in the name of E. I. DuPont and US 5,865,823 , filed on Nov. 6, 1996 in the name of Curro. Other breathable backsheets including nonwoven webs and apertured formed films are described in U.S. Pat. No. 5,571,096 issued to Dobrin et al. on Nov. 5, 1996.

The backsheet 26, or any portion thereof, may be elastically extensible in one or more directions. In one embodiment, the backsheet 26 may comprise a structural elastic-like film ("SELF") web. A structural elastic-like film web is an extensible material that exhibits an elastic-like behavior in the direction of elongation without the use of added elastic materials. SELF webs suitable for the present invention are more completely described in U.S. Pat. No. 5,518,801 entitled Web Materials Exhibiting Elastic-Like Behavior, which issued to Chappell, et al. on May 21,1996.

The backsheet 26 may be joined to the topsheet 24, the absorbent core 28 or any other element of the diaper 20 by any attachment means known in the art. For example, hot melt adhesives applied about the portions of the peripheral edges can be sufficient to join the topsheet and backsheet to one another.

The topsheet 24 is preferably positioned adjacent the body surface 47 of the absorbent core 28 and may be joined thereto and/or to the backsheet 26 by any attachment means known in the art. The topsheet 24 is preferably compliant, soft feeling, and non-irritating to the wearers skin. Further, at least a portion of the topsheet 24 is liquid pervious, permitting liquid to readily penetrate through its thickness. A suitable topsheet 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers.

Any portion of the topsheet 24 may be coated with a lotion as is known in the art. Examples, of suitable lotions include those described in U.S. Pat. Nos. 5,607,760 entitled "Disposable Absorbent Article Having A Lotioned Topsheet Containing an Emollient and a Polyol Polyester Immobilizing Agent" which issued to Roe on Mar. 4,1997; U.S. Pat No. 5,609,587 entitled "Diaper Having A Lotion Topsheet Comprising A Liquid Polyol Polyester Emollient And An Immobilizing Agent" which issued to Roe on Mar. 11, 1997; U.S. Pat. No. 5,635,191 entitled "Diaper Having A Lotioned Topsheet Containing A Polysiloxane Emollient" which issued to Roe et al. on Jun. 3,1997; and U.S. Pat. No. 5,643,588 entitled "Diaper Having A Lotioned Topsheet" which issued to Roe et al. on Jul. 1, 1997.

The absorbent core 28 may comprise any absorbent material which is generally compressible, conformable, non-irritating to the wearer's skin, and capable of absorbing and retaining liquids such as urine and other certain body exudates. The absorbent core 28 may be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt. Examples of other suitable absorbent materials include creped cellulose wadding; melt-blown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The diaper 20 can also comprise at least one elastic waist feature 34 that helps to provide improved fit and containment. The elastic waist feature 34 is generally intended to elastically expand and contract to dynamically fit the wearer's waist. The elastic waist feature 34 preferably extends at least longitudinally outwardly from at least one waist edge 62 of the absorbent core 28 and generally forms at least a portion of the end edge 52 of the diaper 20. Disposable diapers are often constructed so as to have two elastic waist features, one positioned in the first waist region 36 and one positioned in the second waist region 38. Further, while the elastic waist feature 34 or any of its constituent elements may comprise one or more separate elements affixed to the diaper 20, the elastic waist feature 34 may be constructed as an extension of other elements of the diaper 20, such as the backsheet 26, the topsheet 24, or both the backsheet 26 and the topsheet 24. The elastic waist feature 34 may be constructed in a number of different configurations including those described in U.S. Pat. No. 4,515,595 issued to Kievit et al. on May 7, 1985; U.S. Pat. No. 4,710,189 issued to Lash on Dec. 1, 1987; U.S. Pat. No. 5,151,092 issued to Buell on Sept. 9, 1992; and U.S. Pat. No. 5,221,274 issued to Buell on Jun. 22, 1993. Other suitable waist configurations may include waistcap features such as those described in U.S. Pat. No. 5,026,364 issued to Robertson on Jun. 25,1991 and U.S. Pat. No. 4,816,025 issued to Foreman on Mar. 28, 1989.

The diaper 20 may also include a fastening system 40. In particular, tape-type diapers have a fastening system. The fastening system 40 preferably maintains the first waist region 36 and the second waist region 38 in an overlapping configuration so as to provide tension about the circumference of the waist opening of the diaper 20 to hold the diaper 20 on the wearer. The fastening system 40 preferably comprises tape tabs and/or hook and loop fastening components, although any fastening means known in the art is generally acceptable.

In pant-type diapers, opposing sides, i.e., longitudinal edge portions of first waist region 36 and second waist region 38, of the garment are seamed or welded to form a pant, as is known in the art. This allows the article to be used as a pull-on type diaper, such as a training pant.

The diaper 20 can also comprise side panels 30. The side panels 30 may be elastic or extensible to provide a more comfortable and contouring fit by initially conformably fitting the diaper 20 to the wearer and sustaining this fit throughout the time of wear well past when the diaper 20 has been loaded with exudates since the elasticized side panels 30 allow the sides of the diaper 20 to expand and contract. The side panels 30 may be constructed in any suitable configurations as known in the art. Examples of diapers with elasticized side panels are disclosed in U.S. Pat. No. 4,857,067, entitled Disposable Diaper Having Shirred Ears issued to Wood, et al. on Aug. 15, 1989; U.S. Pat. No. 4,381,781 issued to Sciaraffa, et al. on May 3, 1983; U.S. Pat. No. 4,938,753 issued to Van Gompel, et al. on Jul. 3, 1990; the herein before referenced U.S. Pat. No. 5,151,092 issued to Buell on Sep. 9,1992; and U.S. Pat. No. 5,221,274 issued to Buell on Jun.22, 1993; U.S. Pat. No. 5,669,897 issued to LaVon, et al. on Sep. 23, 1997 entitled Absorbent Articles Providing Sustained Dynamic Fit; U.S. Patent application Ser. No.08/155,048 entitled Absorbent Article With Multi-Directional Extensible Side Panels filed Nov. 19, 1993 in the names of Robles, et al.

The diaper 20 preferably further includes leg cuffs 32 which provide improved containment of liquids and other body exudates. Leg cuffs may also be referred to as legseals, side flaps, barrier cuffs, or elastic cuffs. U.S. Pat. No. 3,860,003 describes a disposable diaper which provides a contractible leg opening having a side flap and one or more elastic members to provide an elasticized leg cuff (a gasketing cuff). U.S. Pat. Nos. 4,808,178 and 4,909,803 issued to Aziz et al. on Feb. 28, 1989 and Mar. 20, 1990, respectively, describe disposable diapers having "stand-up" elasticized flaps (barrier cuffs) that improve the containment of the leg regions. U.S. Pat. Nos. 4,695,278 and 4,795,454 issued to Lawson on Sep. 22, 1987 and to Dragoo on Jan. 3, 1989, respectively, describe disposable diapers having dual cuffs, including gasketing cuffs and barrier cuffs.

In prior art diapers longitudinal edges 50 were left unfinished, that is, the edges of the component webs, such as the topsheet 24 and backsheet 26, were left exposed. Although functional, this configuration leaves the diaper with an unfinished look. Attempts to make the diaper more garment-like by finishing the longitudinal edges 50 have been attempted in the art. One known technique is edge folding and seaming, particularly on a curved "hourglass" shaped edge; however, this technique requires complex equipment, and, at best, would only slightly improve the appearance of the edge 50. Another known technique is the addition of separate, discrete portions of edge-finishing materials. This technique involves the cutting, accurate placement and then folding of materials on high-speed equipment. Such technique is described in co-pending, commonly-assigned patent application entitled "Edge Seal for Absorbent Article and Method for Making", U.S. Serial No. 10/262,459, Publication No. 2003/0088227A1, filed on October 1, 2002 to Schneider et al. While such technique provides for a finished cuff, it requires complex folding equipment and additional edge cuff material than that of the present invention. The article of the present invention is an improved diaper having a finished edge cuff. Yet another known technique is the addition of separate, discrete portions of edge-finishing materials being sandwiched between two or more outer material layers (e.g., backsheet and topsheet). While such technique provides for a semi-finished cuff, positioning of the softer edge-finishing material between two outer layers which are not as soft is less desirable. Furthermore, when the edge-finishing material is positioned between the topsheet and backsheet, the finished-look of said material is less noticeable to the consumer/caregiver because of the translucent or opaque properties of the topsheet and backsheet, respectively.

The finished edge cuff comprises an edge band 60 that is joined to a portion of longitudinal edge 50. Edge band 60 preferably comprises a soft, pliable, non-irritating nonwoven material, and is discrete. Edge band can be extensible, and can be elastic or highly elastic, or rendered elastic or highly elastic. In general, materials described as suitable for the topsheet 24 are also suitable materials for use as the edge band 60. By discrete is meant that the edge band 60 is not applied as a continuous member to the entire length of longitudinal edge 50 during diaper manufacture, thereby extending the entire longitudinal length of the diaper 20. Therefore, one advantage of the present invention is edge band 60 need only be joined to longitudinal edge 50 along portions defining the leg opening when diaper 20 is worn. This represents a significant material cost savings. Therefore, each edge band 60 comprises a discrete edge band 60 that is joined to the diaper 20 in a non-wrapping configuration such that it is substantially attached to one surface (e.g., backsheet, topsheet) of at least a portion of the longitudinal edge 50 of the diaper 20 to give the leg opening of diaper 20 a finished, seamed, appearance. Preferably each edge band 60 has dimensions such that when the diaper 20 is worn, the edge band 60 completely encircles the portion of longitudinal edge that defines the leg opening.

Accordingly, in one embodiment, the present invention can be described as a disposable diaper (tape-type or pant-type) comprising a topsheet, a backsheet, and an absorbent core disposed between the topsheet and said backsheet, the topsheet and backsheet defining a periphery and longitudinal edges, portions of the longitudinal edges defining leg openings when the diaper is worn, and an edge band joined to the longitudinal edges at least at the portions defining leg openings when the diaper is worn. In one embodiment the edge bands are joined to the longitudinal edges only at the portions defining the leg openings. In one embodiment the edge bands do not extend the entire length of the longitudinal edge of the diaper.

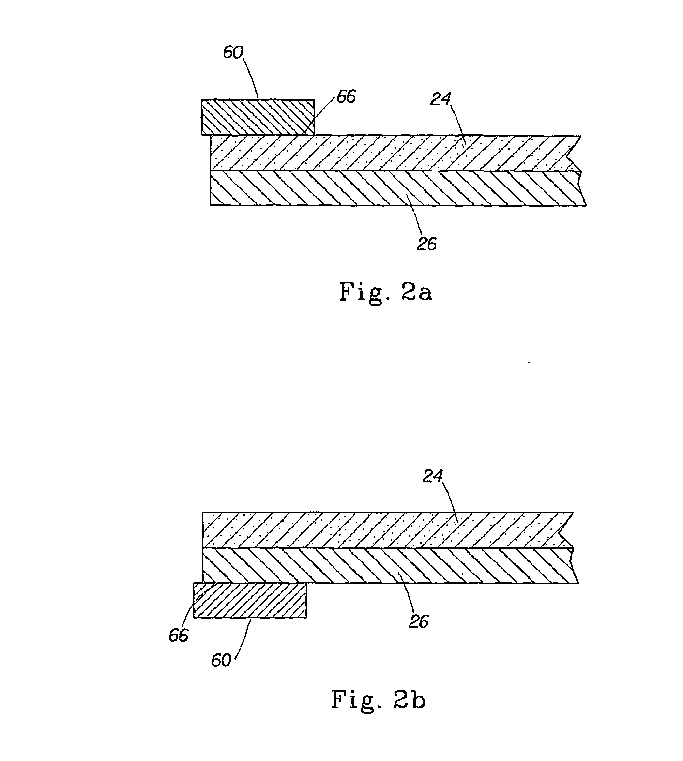

Edge band 60 may be joined to the longitudinal edge 50 of the diaper in a variety of ways. For example, as shown in FIG. 2a, edge band 60 may be joined to the distal end of the inner surface of topsheet 24. Edge band 60 may be joined in a variety of method including, but not limited to, adhesive bonding. For example, hot melt adhesive 66 can be applied to portions of the longitudinal edge (e.g., topsheet 24, backsheet 26) and/or edge band 60 prior to placement of edge band 60 onto said longitudinal edge. If necessary, heat and pressure can be applied as well to ensure sufficient joining of the edge band 60. In an alternative embodiment, as shown in FIG. 2b, edge band 60 may be joined to the distal end of the outer surface of backsheet 26.

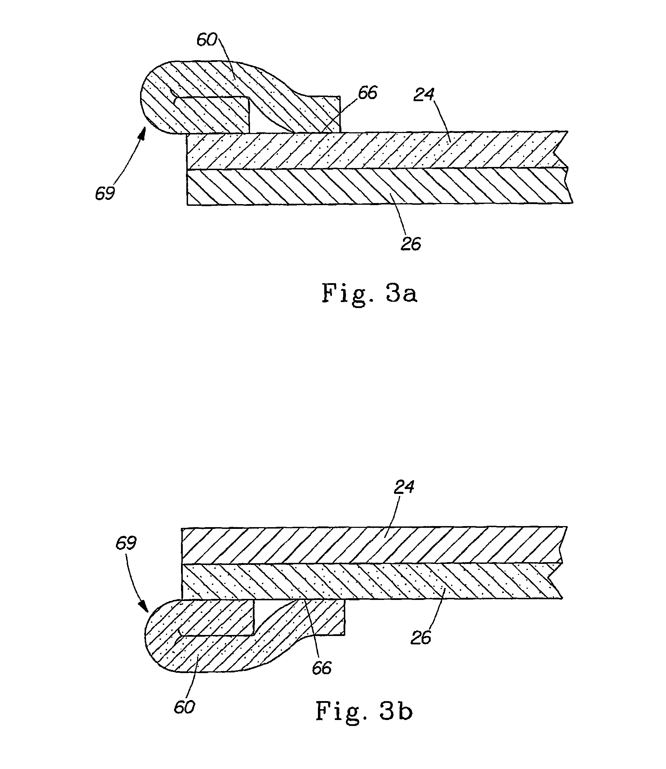

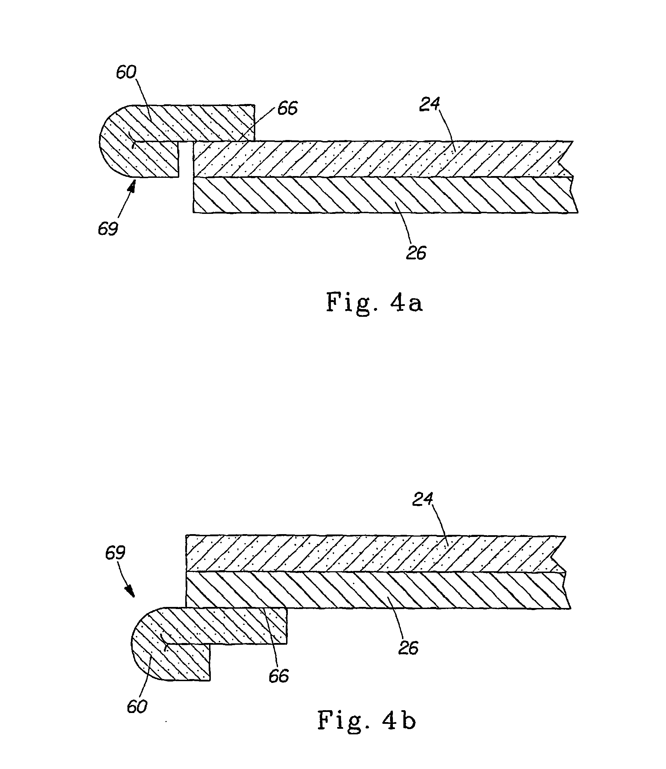

In yet another embodiment, as shown in FIGS. 3a and 3b, edge band 60 may include a fold 69. Said fold 69 may be used to increase the actual and apparent thickness of said edge band such that the consumer/caregiver further appreciates the existence of a finished cuff. Furthermore, the incorporation of fold 69 provides the additional following benefits: (a) any unsightly ends of the longitudinal edges are less noticeable due to the increased visual area of said fold and (b) the increased thickness may provide greater structural integrity in said edge band. The distal end of fold 69 may be joined to the inner surface of topsheet 24 (as shown in FIG. 3a) or the outer surface of backsheet 26 (as shown in FIG. 3b). Alternatively, the distal end of fold 69 may not be joined to the longitudinal edge and thus remain outboard of topsheet 24 (as shown in FIG 4a) or backsheet 26 (as shown in FIG. 4b).

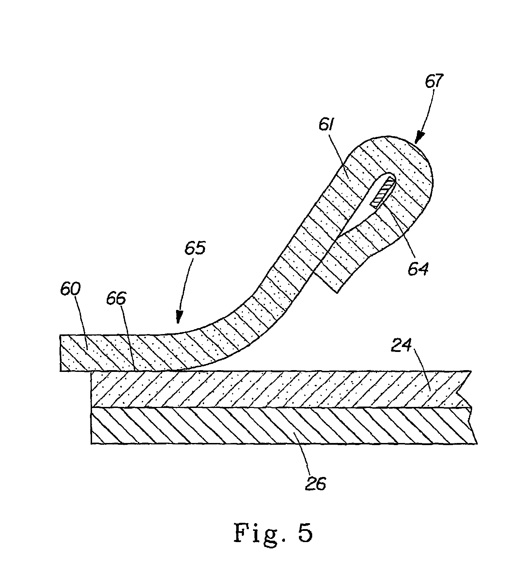

In yet another embodiment, as shown in FIG. 5, a portion of edge band 60 can be extended to form an integrated barrier cuff portion 61, which can replace, or complement elasticized leg cuffs 32. Barrier cuff portion 61 is joined to edge band 60 at a proximal portion 65, but is unattached to topsheet 24 at a distal portion 67. In a preferred embodiment, distal portion 67 comprises an elastic component such as elastic strand 64, or other elastic components as are known in the art of elasticized leg cuffs including barrier leg cuffs. Thus, when in use, elasticized distal portion 67 can "stand up" to form a barrier cuff, as is known in the art of barrier cuffs.

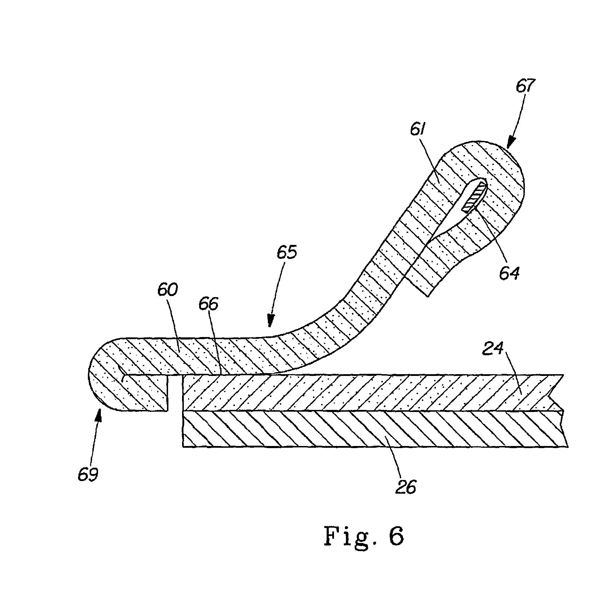

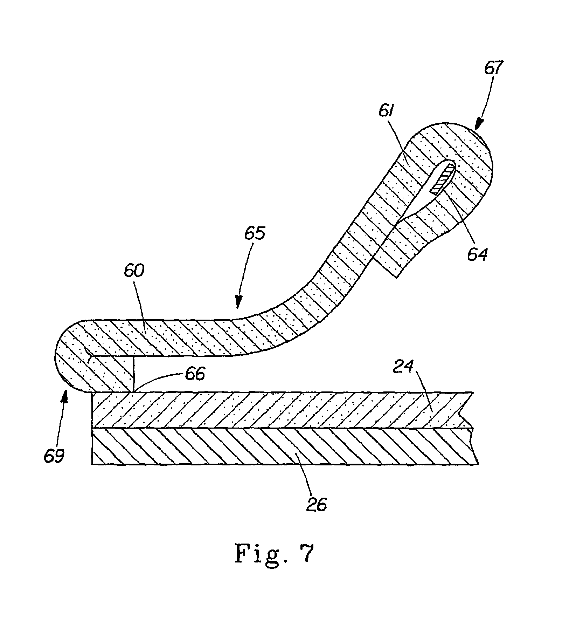

In yet another embodiment, as shown in FIGS. 6 and 7, a portion of edge band 60 can be extended to form an integrated barrier cuff portion 61 (similar to FIG. 5) and also include a fold 69. Furthermore, said fold 69 may be positioned outboard of said topsheet 24 (as shown in FIG. 6; or backsheet 26 not shown) or joined to inner surface of topsheet 24 (as shown in FIG. 7; or outer surface of backsheet 26 not shown).

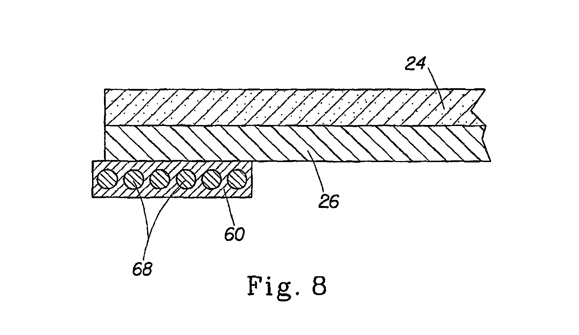

Edge band 60 may be longitudinally elastic. For example, as shown in FIG. 8, edge band 60 may comprise elastomeric materials. Furthermore, edge band 60 may be affixed to longitudinal edge 50 while in a stretched, elongated condition. Once tension is released from edge band 60, it can then contract longitudinally to form gathers along the leg opening of the diaper. The gathers give the diaper leg opening a soft, plush look and feel. Elastomeric properties can be achieved by the use of an elastomeric nonwoven material for example, or by the addition of elastic strands 68 to edge band 60. Furthermore, it may be desirable to provide elastic strands 68 by way of extruding said strands in situ within the diaper manufacturer process. Such extruded elastic strands, and the process of manufacturing, are described in following commonly-assigned patent applications. More specifically, apparatuses for applying elastomeric strands in a longitudinal direction are described in US 7,028,735 entitled "Method and Apparatus for Producing Elastomeric Nonwoven Laminates" filed on June 2, 2003 and in US 7,222,654 entitled "Apparatus for Producing Elastomeric Nonwoven Laminates" filed on April 30, 2004. Apparatuses for applying elastomeric strands in a transverse direction, an angle from the longitudinal direction, or in a curvilinear fashion are described in US 7,201,822 entitled "Method of Placing Material Transversely on a Moving Web" filed on February 13, 2004. Apparatuses for applying elastomeric strands in the longitudinal direction, an angle from the longitudinal direction or in a curvilinear fashion are described in US 7,169,228 entitled"Extrusion Applicator Having Linear Motion Operability" filed on April 29, 2004; and in US 7,097,710 entitled "Extrusion Applicator Having Rotational Operability" filed on April 29, 2004.

### APPARATUS AND METHOD OF MAKING

The edge bands 60 can be affixed to the absorbent article by hand, such as by gluing in position while the article is in a flat, extended configuration. However, such a process is time and labor intensive, and is not feasible for commercial production of absorbent articles. Therefore, the apparatus and method disclosed herein is preferred for commercially viable production of absorbent articles having an edge band 60, and in particular a discrete edge band 60 (i.e., an edge band 60 that not extend the entire length of the longitudinal edge 50 of the diaper).

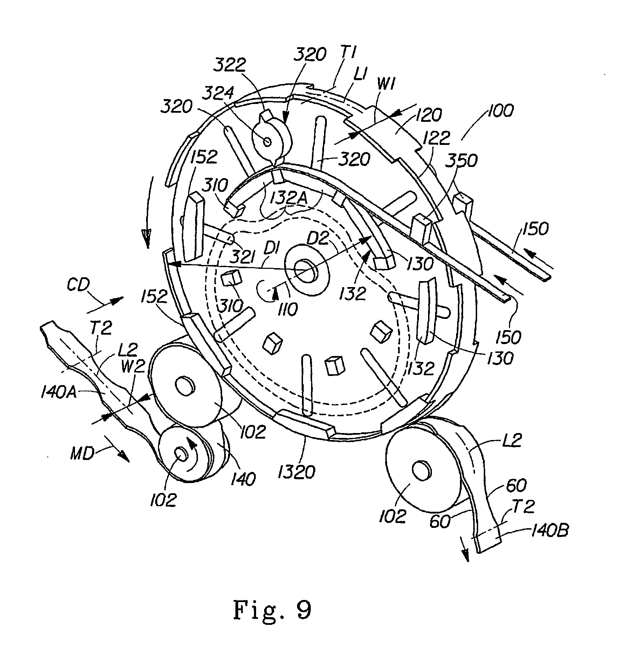

FIG. 9 shows a schematic representation of an apparatus 100 for making an absorbent article having an edge band 60. To process absorbent articles in a commercially viable manner, the apparatus is designed to process materials supplied from continuous webs. However, since in a preferred embodiment the edge band 60 is affixed as a discrete edge band segment (i.e., not a continuous web), the apparatus 100 is designed to process an edge band web material supplied from a continuous web to produce a discrete edge band 60 affixed at spaced apart locations to portions of the longitudinal edge of a continuous web of material that eventually will comprise the chassis of a diaper.

In a commercially viable embodiment edge bands 60 are affixed to both longitudinal edges 50 of diaper 20 simultaneously. Therefore, although the apparatus and process are described below with respect to one edge band web 150 forming one edge band 60, it should be appreciated that the apparatus 100 preferably is configured substantially symmetrically, such that two continuous edge band webs 150 are simultaneously supplied to and processed by the apparatus 100 shown in FIG. 9. Therefore, the description of the apparatus with respect to FIG. 9, for example, is directed to the operation of the apparatus with respect to the side of the apparatus visible in FIG. 9. But the apparatus 100 is two-sided, such that both webs 150 shown in FIG. 9 are simultaneously processed into edge bands 60 of the finished article.

To accomplish the task of applying edge band 60 in a spaced relationship to a continuous web of material being processed at high speed, the apparatus 100 is a rotary apparatus wherein certain operative surfaces rotate about a common axis 110 in a continuous, repeating process, as described herein. In general, the apparatus 100 works on the principle of differential surface path lengths of circumferential (or peripheral) surfaces. That is, as the radial distance from the axis 110 increases, the circumference of a circle associated with the radial distance also increases. The apparatus 100 has at least two operative surfaces, referred to a "web surfaces" herein because the surfaces are designed to handle web materials in a continuous cyclic process.

A first web surface 120 is disposed at a first radial distance D1 from axis 110. The distance D1 can be, as shown in FIG. 9, a fixed distance (i.e., first web surface 120 defines a circular configuration when viewed in profile, that is, parallel to axis 110), but can be a variable distance (i.e., first web surface 120 defines a polygonal configuration when viewed in profile, that is, parallel to axis 110). First web surface 120 is preferably continuous, such as the circumferential surface of a right circular cylinder (D1 is fixed and constant) or the peripheral surface of a right polygonal cylinder (D1 varies from a minimum measured to the center of each polygonal segment to a maximum at the vertex of the angle formed by adjacent polygonal segments). Regardless of the particular configuration of first web surface 120, in a preferred embodiment any given point on first web surface 120 remains at a constant distance D1 from axis 110 during the operation of apparatus 100.

First web surface 120 has a longitudinal axis L1 and a transverse axis T1 perpendicular to longitudinal axis L1. First web surface 120 also has a width W1 measured parallel to the transverse axis T1. Width W 1 can be constant or varying.

A portion of first web surface 120 supports a continuous web 140 having a longitudinal axis L2 oriented in the machine direction MD, and a transverse axis T2 perpendicular to longitudinal axis L2. Continuous web 140 also has a width W2 measured in the cross-direction CD, parallel to the transverse axis T2 (perpendicular to the MD), and width W2 can be constant or varying. In operation, continuous web 140 can be guided adjacent to, and preferably in contact with, a portion of first web surface 120, which is designed to support web 140 during the portion of the process in which portions of edge band web material 150 are joined thereto to become edge bands 60 as discussed more fully below. In a preferred embodiment, during processing as described herein, the longitudinal axis L2 of web 140 is aligned parallel and adjacent to longitudinal axis L 1 of first web surface 120.

Continuous web 140 can be a portion of the absorbent article in process. That is, continuous web 140 can be a laminate web comprising multiple layers of component materials, such as diaper chassis materials, in the process of being assembled into a finished absorbent article, as is known in the art of commercial disposable diaper manufacture. For example, continuous web 140 can be a liquid impermeable backsheet material, or a nonwoven topsheet material. In a preferred embodiment, continuous web 140 is a laminate comprising a continuous web of backsheet and topsheet material joined together as is known in the art of diaper manufacture. In a preferred embodiment, as shown on incoming continuous web 140A in FIG. 9, portions of longitudinal edges of continuous web 140 are cut, or "notched" to form curvilinear notched portions that form an "hourglass" shaped chassis in the finished article. In an alternative embodiment (not shown), the incoming continuous web 140A is not pre-cut or pre-notched prior to the guide rollers 102 that are positioned prior to edge band application, rather said guide rollers could include a rotary die cutter to form curvilinear notched portions that form an "hourglass" shaped chassis in the finished article immediately prior to the discrete edge band application. In this alternative embodiment, mis-matching of the notches with the shape of the discrete edge bands that would normally be caused by web tracking/phasing variations are minimized. In yet another alternative embodiment (not shown), the incoming continuous web 140A is not pre-cut or pre-notched prior to the guide rollers 102 that are positioned prior to edge band application, rather said notches are cut after and in close proximity to the application of the discrete edge bands. Lastly, a plurality of discontinuous and spaced apart absorbent core elements (not shown in FIG. 9) can be disposed between the continuous web of backsheet and topsheet materials.

Continuous web 140 can be guided adjacent to web surface 120 by any suitable means. In one embodiment, as shown in FIG. 9, guide rollers 102 are operatively positioned such that continuous web 140 is urged against a portion of web surface 120, which correspondingly supports continuous web 140 for a portion that can be described as a defined arc length of first web surface 120. If first web surface 120 is not at a constant distance from axis 110, i.e., the web surface 120 is not circular in shape when viewed parallel to axis 110, then guide rollers 102 can be correspondingly operatively positioned. For example, in one embodiment, each guide roller 102 can itself be spaced a distance from axis 110 equal to or greater than the maximum distance D1. In another embodiment guide rollers 102 can be biased, such as by spring loading, to provide constant pressure against web surface 120 even as its radial distance from axis 110 changes.

In operation, continuous web 140 is guided in the direction shown by the arrows associated therewith, as the apparatus 100 rotates about axis 110 in the direction shown by the arrows associated therewith (counter-clockwise as viewed in FIG. 9). The linear, i.e., tangential speed of web surface 120 is essentially the same as, and preferably exactly the same as, the linear speed of continuous web 140, such that during the portion of the process in which continuous web 140 is disposed adjacent first web surface 120, there is no relative motion between continuous web 140 and first web surface 120.

In one embodiment, first web surface 120 can have a varying width W1 defining spaced apart notches 122. In a preferred embodiment, the varying width W1 defines a plurality of regularly spaced, opposing notches, as shown in FIG. 9. In operation of a preferred embodiment of apparatus 100 the notches correspond to corresponding notched portions of continuous web 140 to which edge bands 60 are applied. That is, notched portions of continuous web 140 line up adjacent to respective notched portions of first web surface 120 when continuous web 140 is disposed adjacent to first web surface 120. Thus, edge bands 60 can be discrete members that provide a finished edge to the leg openings defined by the concave portions of an hourglass-shaped diaper chassis. However, it is not necessary that first web surface 120 exhibit such notches. In one embodiment of apparatus 100, for example, portions of the width W2 of web 140 are sufficiently greater than a constant width W1 of web surface 120, so that the edge bands 60 can be affixed to such portions (i.e., overhanging edges of web 140).

Edge bands 60 are discrete elements joined to a portion of each longitudinal edge 50 of diaper 20. However, in a commercially viable process, it is necessary that edge bands 60 be processed from a continuous web material preferably supplied as roll stock or festooned for substantially continuous processing as a web. To facilitate web joining of a relatively short discrete member to a relatively longer continuous member, the apparatus 100 of the present invention has a second web surface 130 that is at a second radial distance D2 from axis 110. Second web surface 130 can be comprised of a plurality of edge band placers 132, each of which are preferably identical to one another. Each edge band placer 132 is designed to convey a discrete edge band portion 152, which, once joined to continuous web 140 becomes edge band 60. Thus, edge band placers 132 receive a continuous web of edge band material 150 and subsequently facilitate control of edge band material 150 as it is cut to make a discrete edge band portion 152 having a predetermined length and which is translated to a predetermined position and joined to a predetermined portion of continuous web 140 to form an edge band 60 of the present invention. The predetermined locations are spaced apart, and correspond to the portion of the chassis longitudinal edges 50 that define at least portions of a leg opening when the finished garment is worn.

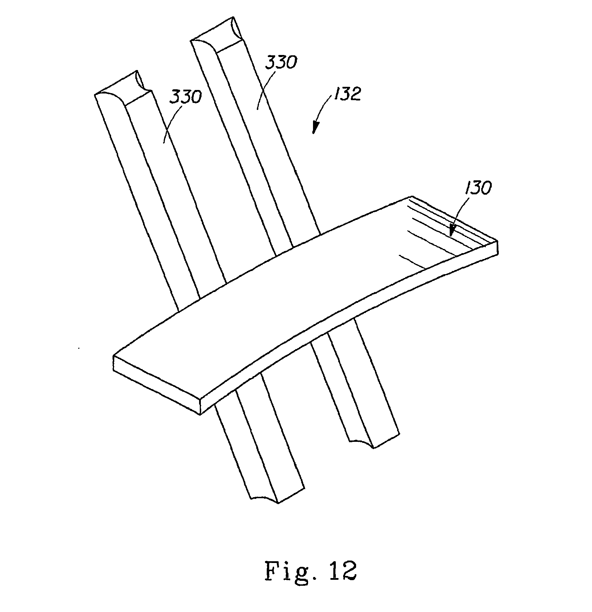

Each edge band placer 132 defines a portion of second web surface 130 that can be substantially flat in profile (i.e., as viewed perpendicular to axis 110), or each can define a second web surface 130 that makes an arc segment in profile as shown in FIG. 12. The actual profile of edge band placers 132 preferably matches the profile of first web surface 120. That is, the arc radius defined by the second web surface 130 of edge band placers 132 is preferably identical to the arc radius defined by the first web surface 120.

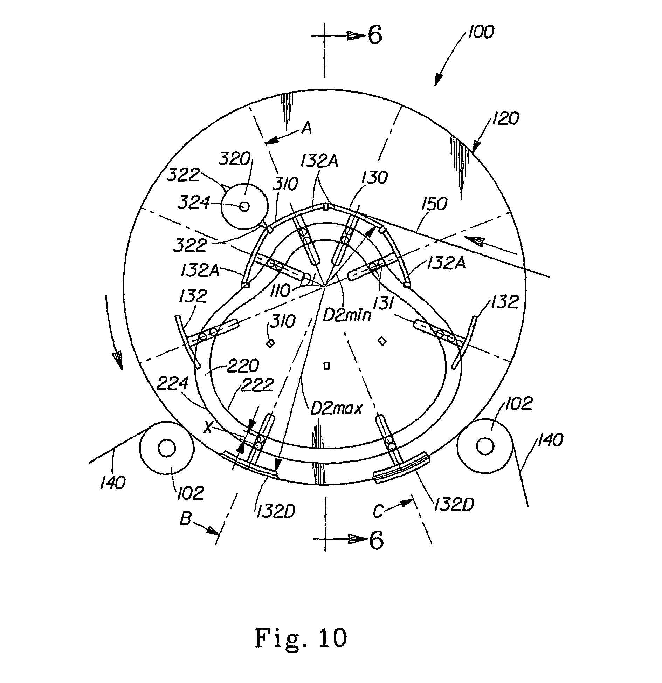

Each edge band placer 132 occupies a position at a variable distance D2 from axis 110. Each edge band placer 132 can translate, i.e., move, from a first position that is a minimum radial distance D2min from axis 110 to a second position that is a maximum radial distance D2max from axis 110, as shown in FIG. 9. Three edge band placers 132 shown as segments 132A in FIGS. 9 and 10 are at minimum radial distance D2min. In the embodiment shown in FIG. 9, the apparatus 100 rotates as shown by the arrow associated therewith (i.e., in a counter-clockwise manner as viewed in FIG. 9), and as it rotates, edge band placers 132 are translated from minimum radial distance D2min in a direction away from axis 110 until they reach a maximum radial distance D2max. An edge band placer 132 in this position is shown as segment 132D in FIGS. 9 and 10.

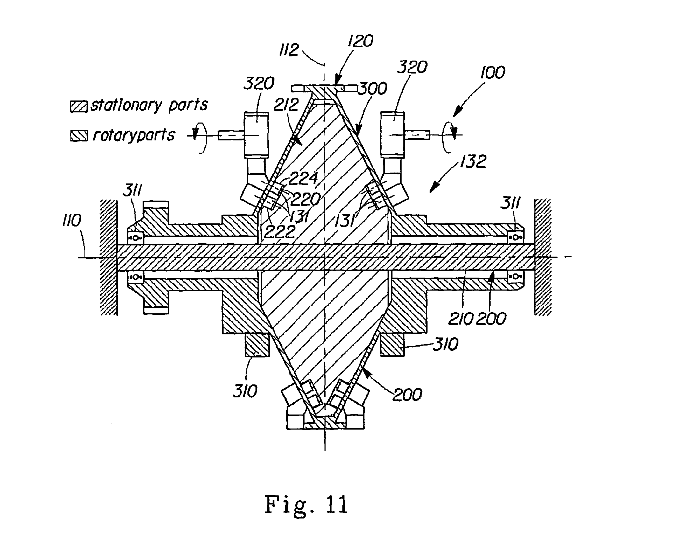

In addition to translating in a direction radially outwardly from (or inwardly toward) axis 110, edge band placers 132 preferably translate in a direction parallel to axis 110. That is, as can be seen best in the cross section of FIG. 11, edge band placers 132 receive continuous edge band web 150 at a maximum distance from line of symmetry 112, and move toward the line of symmetry 112 when moving away from axis 110. In this manner, both continuous webs 140 and 150 can be processed from their respective supply packages generally to one another.

In the embodiment shown in FIG. 9, when segments 132 are at the maximum radial distance D2max, the second web surface 130 and first web surface 120 are at a substantially equal radial distance from axis 110. By "substantially equal" is meant that, for practical purposes, the two surfaces are the same distance from axis 110, but the term is not meant to convey mathematical exact equality. In some processes it may be desirable to have a difference in radial distance between the first and second web surfaces when edge band placers 132 are at the maximum radial distance D2max.

Edge band placers 132 are translated from a first position, at which position edge band web 150 is operatively disposed thereon, to a second position, at which position the edge band portion 152 is affixed to the continuous web 140, that is, to what will eventually be the longitudinal edges of a disposable diaper, and back to the first position in a continuous, repeating process. By "operatively disposed" thereon is meant that the edge band web 150 is received by and disposed on edge band placer 132 in a generally flattened, extended position and orientation with respect to the edge band placer 132, such that once edge band web 150 is cut into discrete edge band portion 152 and edge band placer 132 begins translating it to its respective second position, the edge band portion 152 is accurately positioned for affixing to the continuous web 140 to form a completed edge band 60.

The apparatus and process are now discussed in more detail with reference to a particular method of operation with respect to FIGS. 9, 10 and 11. FIG. 10 is a schematic representation of the apparatus 100 as viewed parallel to axis 110. FIG. 11 is a cross-sectional view of apparatus 100.

As one can see with reference to FIGS. 9 and 10, the apparatus 100 permits continuous webs 140, 150 to be processed into an article of the present invention having discrete (i.e., non-continuously applied) edge bands 60. That is, although each edge band could be supplied as discrete "patches" or sheets of material, and applied by hand or by automatic placement equipment onto the second web surface 130 of each edge band placer 132 when it is at its first position D2min, in a preferred embodiment, edge band web material 150 is supplied as a continuous web of material, such as a relatively narrow strip of material (e.g., non-woven). While the general principle of operation can be practiced in various ways, the following description is intended to be a nonlimiting example of a currently preferred apparatus.

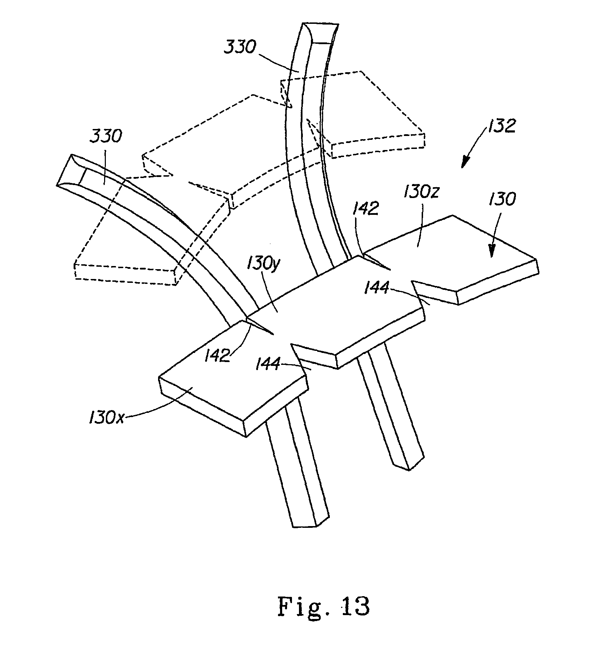

Apparatus 100 comprises a stationary portion 200 and a rotating portion 300. Stationary portion 200 comprises a mounting shaft 210 that is parallel to, and coaxial with axis 110 of apparatus 100. Affixed to shaft 210 is cam plate 212. Cam plate 212 comprises a cam track 220, which itself comprises an inner cam track surface 222 and an outer cam track surface 224. Rotating portion 300 can be mounted on suitable bearings 310, such as ball bearings, and rotates about shaft 210. First surface 120 forms an outer periphery of rotating portion 300. Rotating portion 300 has a plurality of slots 320 (shown in FIG. 9) and guides 330 (shown in FIGS. 12 and 13) that serve to facilitate linear motion of edge band placers 132 in a direction radially outwardly from axis 110. Guides 330 can be linear slide bearings, for example. Each edge band placer 132 is operatively attached through a respective slot 320 to a pair of cam followers 131 disposed in operative relationship within cam track 220. Therefore, as rotating portion 300 is rotated, each edge band placer 132 is forced by the cam followers 131 in cam track 220 to follow a complex path, rotationally about and away from axis 110 and toward plane of symmetry 112, or vice-versa, depending on where the edge band placer is in the cycle of operation. Starting from the position identified as 132A, the rotation of rotating portion 300 forces edge band placers 132 to translate relative to axis 110, which has the effect of moving portions of second web surface 130 from minimum distance D2min to maximum distance D2max with respect to axis 110 and back in a repeating cycle.

In a preferred embodiment of apparatus 100, when at minimum distance D2min, each edge band placer 132 is separated from adjacent edge band placers 132 by a stationary anvil 310 that provides a cutting surface for a web cutting device, such as rotary knife 320. Stationary anvil 310 is stationary with respect to rotating portion 300, and can be, for example, a permanently affixed (to rotating portion 300), relatively rigid member having a relatively smooth cutting surface.

Rotary knife 320, mounted separately from rotating portion 300, can have a plurality of cutting blades 322 that operate in timed sequence with rotary portion 300 such that cutting blades 322 sever edge band web material 150 thereby forming discrete edge band portions 152. Other cutting methods can be utilized, such as laser cutting, high-speed fluid cutting, saw blade cutting, and the like. In the embodiment shown in FIGS. 9 and 10, rotary knife 320 is rotatable upon its axis of rotation 324, which is parallel to axis 110 of apparatus 100. The rotary knife 320 rotates in a timed relationship with rotary portion 300, such that the blade cuts continuous web 150 at a location corresponding to an area between adjacent edge bands placers 132, while the respective adjacent edge band placers are each at the position designated as 132A.

In another embodiment, web 150 can be perforated across its width in a regularly repeating pattern of spaced apart, longitudinally extending perforations (e.g., as is common in rolled toilet tissue). In this configuration, the distance between lines of perforation would correspond to the long dimension of edge band placer 132. As each edge band placer 132 begins translating from the first position D2min to the second position, D2max, the lines of perforation can tear, thereby rendering each edge band portion 152 discrete and operatively disposed on its respective edge band placer 132.

In operation, therefore, as rotating portion 300 rotates edge band web material 150 is drawn onto second web surface 130 while second web surface is at minimum distance D2min. In FIG. 10, edge band placers 132 designated 132A are shown in the position in which edge band web material 150 is drawn or received onto apparatus 100 as rotating portion 300 rotates about axis 110. To stabilize edge band web material 150 in position on second web surface 130, each edge band placer 132 can have vacuum ports to which a partial pressure is applied. In this manner, edge band web material 150 is held in position on each edge band placer by vacuum, or suction, forces. The back tension applied to edge band web material 150 as it is drawn from its supply package (e.g., roll or festoon), is controlled in conjunction with the forces supplied by the vacuum on edge band placers 132 to ensure edge band web material 150 is drawn onto apparatus 100 in a stable, continuous process. Vacuum can be applied in ways known in the art, such as by suitable arrangements of pumps, tubes, and vacuum ports. Other means known in the art for stabilizing web materials can be used as well. For example, each edge band placer can have a light-tack adhesive applied to the surface 130 such that edge band web material 150 is removably adhered in place when cut into edge band portions 152.

After having been cut by rotary knife 320, discrete edge band portion 152 is disposed on its respective edge band placer 132 in a generally flattened position. Following the sequence of motion counter-clockwise (with respect to FIG. 10), the edge band placer 132 is translated via the interaction of cam followers 131 and cam track 220 outwardly with respect to axis 110 and inwardly with respect to plane of symmetry 112 toward first web surface 120. At its maximum distance from axis 110, which is D2max, second web surface 130 is at a distance from axis 110 that is essentially equal to that of first web surface 120. At this position, edge band web portion 152 is adjacent to and partially overlaps continuous web 140.

Once placed against a longitudinal edge of continuous web 140, edge band portions 152 are joined to continuous web 140 by methods known in the art for assembling components of absorbent articles, particularly disposable absorbent articles. In a preferred embodiment, edge band portions 152 are affixed by adhesive, such as hot melt adhesive. The adhesive can be applied to the continuous web 140 at locations intended to receive the edge band portions 152, or adhesive can be applied to one surface of the edge band web 150 prior to being cut by rotary knife 320. Adhesive can be applied by methods known in the art, including extrusion, spraying, slot coating, or wiping. In one embodiment, adhesive is applied by extruding hot melt adhesive beads from adhesive applicators 350 placed to facilitate adhesive application to edge band web 150. The particular method and location of applying adhesive depends on the type of adhesive used, and its cure rate, and is not considered critical to the present invention. In one embodiment Findley H2031 adhesive, applied by spraying onto edge band web 150 was used.

In one embodiment, the edge band web 150 comprises elastic material, or highly elastic material, and is received onto edge band placers 132 as a tensioned web of material in an extended state. Once applied to the longitudinal edge of continuous web 140, the edge band 60 so formed can remain in an extended, stretched configuration. Once continuous web 140 is cut and formed into a diaper, edge band 60 can relax, contract, and form a soft, gathered, finished cuff about the leg portion of diaper 20. In one embodiment, edge band web 150 comprises nonwoven material comprising elastomeric strands of an elastomeric polymer, such as Kraton®, Lycra®, or Spandex® strands.

In one embodiment, edge band web 150 comprises at least one strand of elastomeric polymer strand(s) enveloped in a fold along one longitudinal edge thereof. The width of edge band web 150, including the edge having enclosed therein an elastomeric strand is adequate to provide not only an edge band, but also the integrated barrier cuff 61 as shown in FIGS. 5-7. Thus, upon folding, edge band portion 152, having an extended width, forms not only edge band 60, but integrated barrier cuff 61.

In a preferred embodiment, edge bands 60 are not straight when applied to the longitudinal edge 50 of diaper 20 (and, therefore, not straight when applied as edge band portion 152 to a longitudinal edge of continuous web 140). In a preferred embodiment, edge bands 60 follow the contour of the concave-shaped portions of a diaper (in a flat-out condition, as shown in FIG. 1) of a diaper having an "hourglass" shape. Therefore, in a preferred embodiment, because edge band web 150 is received as a "straight" web, and edge band segment 152 is therefore received in an "un-curved" configuration, the edge band placers 132 are segmented such that they may articulate. Such articulating segments 130x, 130y and 130z are shown schematically in FIG. 13. In practice, the number of segments can be varied as necessary to achieve the curvature of edge band 60 desired. These segments may be separated by partial slits 142, and V-shaped openings 144. In this embodiment, guides 330 do not follow a completely linear path, but as edge band placer 132 nears D2max, guides 330 deviate from linear, for example by twisting with respect to each other. The exact path of guides 330 is difficult to describe in words, but results in at least one or both of segments 130x and 130z articulating with respect to 130y, for example, such that slit 142 opens up in a V shape, and V-shaped opening 144 closes up, so that the edge band placer 132 forms curve. Said curve in edge band placer 132 follows the contour of longitudinal edge 50 of the finished article, as shown in FIG. 1.

In a preferred embodiment, edge bands 60 may undergo post-application activation to impart additional softness and/or extensibility. With the edge band being located on the inner surface of said longitudinal edges (e.g., topsheet) or the outer surface of the longitudinal edges (e.g., backsheet), post-application activation may be directly applied for maximum utilization and benefit. Additionally, it may be desirable to provide said activation such that the resulting activation lines/corrugations may be substantially perpendicular along the curved longitudinal edges. The processes and techniques for performing post-application activation are described in commonly-assigned U.S. Patent No. 6,764,478, entitled "Disposable Absorbent Garment Having Highly Extensible Leg Openings", to Ashton et al., issued on 7/20/2004.

All documents cited are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

For example, while the present application speaks of applying a discrete edge band on the inner or outer surfaces of the longitudinal edge (e.g., topsheet or backsheet, respectively), one skilled in the art would appreciate that other layers of materials may suffice as either the inner or outer surface (e.g., the barrier leg cuff may extend outwardly and above the topsheet such that the barrier leg cuff may be properly deemed as the outer surface of the longitudinal edge).

## Claims

1. A disposable diaper comprising a topsheet, a backsheet, and an absorbent core disposed between said topsheet and said backsheet, said topsheet and said backsheet defining a periphery and longitudinal edges, portions of said longitudinal edges defining leg openings when said disposable diaper is worn, said disposable diaper **characterized** such that on each longitudinal edge a discrete edge band joined to the inner surface of said longitudinal edges at least at said portions of said longitudinal edges defining leg openings when said disposable diaper is worn, wherein said discrete edge band further having a fold and wherein said discrete edge bands do not extend the entire length of said longitudinal edge of said disposable diaper.

2. The disposable diaper of Claim 1 wherein said discrete edge band joined is affixed to said topsheet.

3. The disposable diaper of Claim 1 wherein said discrete edge bands are joined to each of said longitudinal edges only at said portions of said longitudinal edges defining leg openings.

4. The disposable diaper of Claim 1 wherein said edge discrete band having an elastomeric material.

5. The disposable diaper of Claim 1 wherein said discrete edge band having a nonwoven material.

6. The disposable diaper of Claim 1, wherein said discrete edge band having at least one elastomeric strand.

7. The disposable diaper of Claim 6 wherein said elastomeric strand is an extruded elastic strand.

8. The disposable diaper of Claim 1, wherein said discrete edge band further having a barrier cuff.

9. The disposable diaper of Claim 1 wherein said fold is affixed to said topsheet.

## Patentansprüche

1. Einwegwindel, umfassend eine Oberschicht, eine Unterschicht und einen Absorptionskern, der zwischen der Oberschicht und der Unterschicht angeordnet ist, wobei die Oberschicht und die Unterschicht einen Umfang und Längsränder definieren, wobei Abschnitte der Längsränder Beinöffnungen definieren, wenn die Einwegwindel getragen wird, wobei die Einwegwindel an jedem Längsrand ein separates Randband umfasst, das mit der Innenoberfläche der Längsränder mindestens an den Abschnitten der Längsränder verbunden ist, die Beinöffnungen definieren, wenn die Einwegwindel getragen wird, wobei das separate Randband ferner eine Faltung aufweist und wobei die separaten Randbänder sich nicht über die gesamte Länge des Längsrands der Einwegwindel erstrecken.

2. Einwegwindel nach Anspruch 1, wobei das angebrachte separate Randband an der Oberschicht befestigt ist.

3. Einwegwindel nach Anspruch 1, wobei die separaten Randbänder nur in den Abschnitten der Längsränder, die Beinöffnungen definieren, an jedem der Längsränder angebracht sind.

4. Einwegwindel nach Anspruch 1, wobei das separate Randband ein Elastomermaterial umfasst.

5. Einwegwindel nach Anspruch 1, wobei das separate Randband ein Vliesmaterial umfasst.

6. Einwegwindel nach Anspruch 1, wobei das separate Randband mindestens einen elastomeren Strang aufweist.

7. Einwegwindel nach Anspruch 6, wobei der elastomere Strang ein extrudierter Elastikstrang ist.

8. Einwegwindel nach Anspruch 1, wobei das separate Randband ferner ein Sperrbündchen aufweist.

9. Einwegwindel nach Anspruch 1, wobei die Faltung an der Oberschicht befestigt ist.

## Revendications

1. Couche jetable comprenant une feuille de dessus, une feuille de fond, et une âme absorbante disposée entre ladite feuille de dessus et ladite feuille de fond, ladite feuille de dessus et ladite feuille de fond définissant une périphérie et des bords longitudinaux, des parties desdits bords longitudinaux définissant des ouvertures de jambe lorsque ladite couche jetable est portée, ladite couche jetable comprenant sur chaque bord longitudinal une bande de bord discrète jointe à la surface interne desdits bords longitudinaux au moins au niveau desdites parties desdits bords longitudinaux définissant des ouvertures de jambe lorsque ladite couche jetable est portée, dans laquelle ladite bande de bord discrète a en outre une pliure et dans laquelle lesdites bandes de bord discrètes ne s'étendent pas sur la longueur entière dudit bord longitudinal de ladite couche jetable.

2. Couche jetable selon la revendication 1, dans laquelle ladite bande de bord discrète jointe est attachée à ladite feuille de dessus.

3. Couche jetable selon la revendication 1, dans laquelle lesdites bandes de bord discrètes sont jointes à chacun desdits bords longitudinaux uniquement au niveau desdites parties desdits bords longitudinaux définissant des ouvertures de jambe.

4. Couche jetable selon la revendication 1, dans laquelle ladite bande de bord discrète comprend un matériau élastomère.

5. Couche jetable selon la revendication 1, dans laquelle ladite bande de bord discrète comprend un matériau non tissé.

6. Couche jetable selon la revendication 1, dans laquelle ladite bande de bord discrète a au moins un fil élastomère.

7. Couche jetable selon la revendication 6, dans laquelle ledit fil élastomère est un fil élastique extrudé.

8. Couche jetable selon la revendication 1, dans laquelle ladite bande de bord discrète a en outre un revers formant barrière.

9. Couche jetable selon la revendication 1, dans laquelle ladite pliure est attachée à ladite feuille de dessus.
